# EUROPÄISCHE PATENTSCHRIFT

(11) **EP 3 291 858 B1**
(45) Veröffentlichungstag und Bekanntmachung des Hinweises auf die Patenterteilung: **13.03.2019**
(21) Anmeldenummer: 15723447.7
(22) Anmeldetag: 04.05.2015
(51) Int. Cl.: A61M 5/20

(54) **EINWEGINJEKTOR MIT EINER AUSLÖSEMONTAGEEINHEIT ZUM VEREINFACHTEN ZUSAMMENBAU**
ONEWAYINJECTOR HAVING A TRIGGER ASSEMBLY UNIT FOR EASE OF ASSEMBLY
INJECTEUR À UNE VOIE AVEC UNITÉ DE DÉCLENCHEMENT DE MONTAGE POUR UN ASSEMBLAGE FACILE

(43) Veröffentlichungstag der Anmeldung: 14.03.2018
(73) Patentinhaber: LTS Lohmann Therapie-Systeme AG, 56626 Andernach (DE)
(72) Erfinder: FORGHANI, Sara, 56182 Urbar (DE); HEUSER, Karsten, 53498 Bad Breisig (DE); SCHERR, Sebastian, 56335 Neuhäusel (DE); WORTMANN, Uwe, 35037 Marburg (DE)
(74) Vertreter: Thämer, Wolfgang
(86) Internationale Anmeldenummer: PCT/EP2015/059683
(87) Internationale Veröffentlichungsnummer: WO 2016/177389

(56) Entgegenhaltungen:
- WO-A1-2011/048223
- WO-A1-2015/028393
- DE-A1-102007 032 463
- DE-A1-102008 063 519
- US-A- 3 557 784
- US-A1- 2011 098 647

## Beschreibung

Die Erfindung betrifft einen Einweginjektor mit einem in einem Gehäuse gelagerten, mittels eines Federenergiespeichers belasteten und mittels einer verschiebbaren Auslösevorrichtung entsperrbaren Kolbenbetätigungsstempel, wobei der Kolbenbetätigungsstempel mittels eines im Gehäuse gelagerten Zugstabsabstützbar ist, wobei die Auslösevorrichtung ein Mantelgehäuse umfasst, wobei das Mantelgehäuse eine Oberschale und eine Unterschale umfasst, deren Trennfuge in der Längsrichtung des Einweginjektors orientiert ist, wobei das Mantelgehäuse einen von der kreisförmigen Form abweichenden eckenlosen Querschnitt aufweist, wobei das Gehäuse im Mantelgehäuse verdrehsicher zentriert ist und wobei der Kolbenbetätigungsstempel einen Führungszapfen, einen Stempelteller und einen Kolbenschieber umfasst.

Aus der DE 10 2008 063 519 A1 ist ein derartiger Einweginjektor bekannt. Die Herstellung und Montage dieses Einweginjektors ist aufwendig.

Aus der WO 2015/028393 A1, der WO 2011/048323 A1, der DE 10 2007 032 463 A1, der US 3557784 und der US 2011/0098647 A1 sind Injektoren mit vielen Einzelteilen bekannt. Ihre Montage ist aufwendig.

Der vorliegenden Erfindung liegt die Problemstellung zugrunde, die Herstellung, die Montage und die Handhabung des Einweginjektors zu vereinfachen.

Diese Problemstellung wird mit den Merkmalen des Hauptanspruchs gelöst. Dazu hat der Stempelteller eine dem Führungszapfen abgewandte, konisch ausgebildete Bundfläche, auf der eine kegelstumpfförmig ausgebildete Stützscheibe aufliegt, deren Spitzenwinkel dem Spitzenwinkel der Bundfläche entspricht. Der Zugstab ist u-förmig und streifenförmig ausgebildet und umfasst einen Hauptschenkel, einen Klemmschenkel und einen Umgriffsschenkel. Der Zugstab ist mittels des Klemmschenkels im Gehäuse gelagert. Der Umgriffsschenkel ragt durch einen schlitzartigen Durchbruch des Gehäuses hindurch und liegt an einer Unterseite der Stützscheibe an. Der Zugstab stützt sich an einer eine Panzerung bildenden Gleitplatte eines Auslöserings ab. Der Auslösering umgreift das Gehäuse und ist zwischen einer Sperrflanke und einer Auslöseflanke des Mantelgehäuses angeordnet. Beim Auslösen verschiebt das eine Auslösehülse bildende Mantelgehäuse mittels der Auslöseflanke den Auslösering relativ zum Gehäuse in der Auslöserichtung nach vorne. Der Federenergiespeicher drückt den Stempelteller nach vorne und die Druckscheibe verdrängt den Umgriffshaken des Zugstabs. Der Zugstab gleitet entlang der Gleitplatte nach außen und gibt den Kolbenbetätigungsstempel vollständig frei.

Weitere Einzelheiten der Erfindung ergeben sich aus den Unteransprüchen und den nachfolgenden Beschreibungen schematisch dargestellter Ausführungsbeispiele.
- Figur 1:: Explosionszeichnung eines Einweginjektors;
- Figur 2:: Dimetrische Darstellung des Gehäuses;
- Figur 3:: Kolbenbetätigungsstempel;
- Figur 4:: Zugstab;
- Figur 5:: Auslösering;
- Figur 6:: Stützscheibe;
- Figur 7:: Vormontagegruppe ohne Zylinder-Kolben-Einheit;
- Figur 8:: Längsschnitt der Vormontagegruppe aus Figur 7;
- Figur 9:: Vormontagegruppe und Mantelgehäuse vor der Endmontage;
- Figur 10:: Einweginjektor nach der Montage;
- Figur 11:: Querschnitt des Einweginjektors aus Figur 10;
- Figur 12:: Längsschnitt des Einweginjektors aus Figur 10;
- Figur 13:: Längsschnitt normal zu Figur 12;
- Figur 14:: Einweginjektor nach dem Auslösen;
- Figur 15:: Einweginjektor mit entleerter Zylinder-Kolben-Einheit.

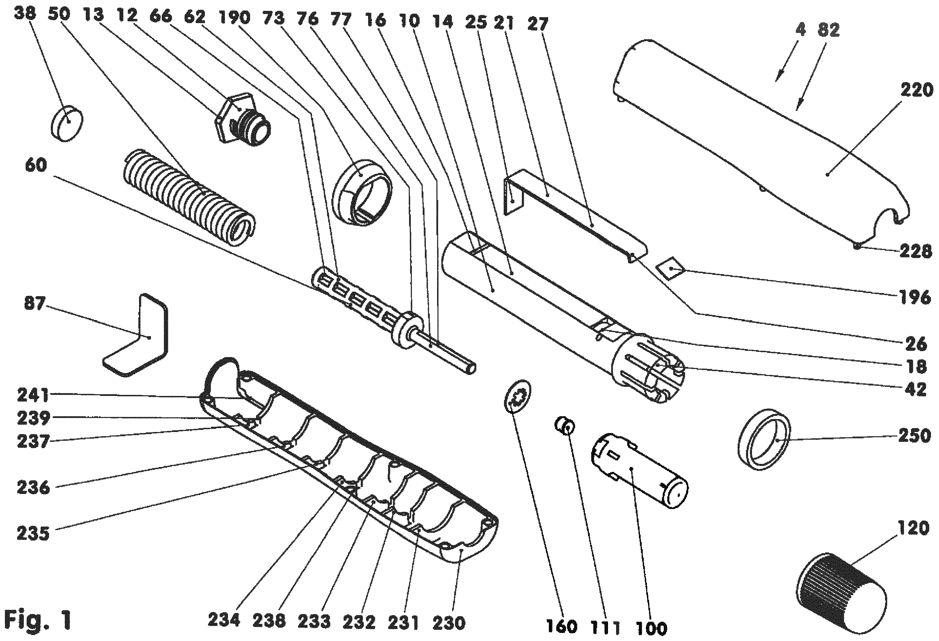

Die Figuren 1 - 15 zeigen einen Einweg- oder Einmalinjektor (4). Derartige Injektoren (4) werden zum einmaligen Einbringen einer Injektionslösung (1) oder eines Lösemittels in die Haut eines Patienten eingesetzt.

Der in der Figur 1 in einer Explosionszeichnung dargestellte Einweginjektor (4) umfasst ein inneres Gehäuse (10), einen Federenergiespeicher (50), einen Kolbenbetätigungsstempel (60), einen Zugstab (21), einen Auslösering (190), eine Zylinder-Kolben-Einheit (100) und ein Mantelgehäuse (82).

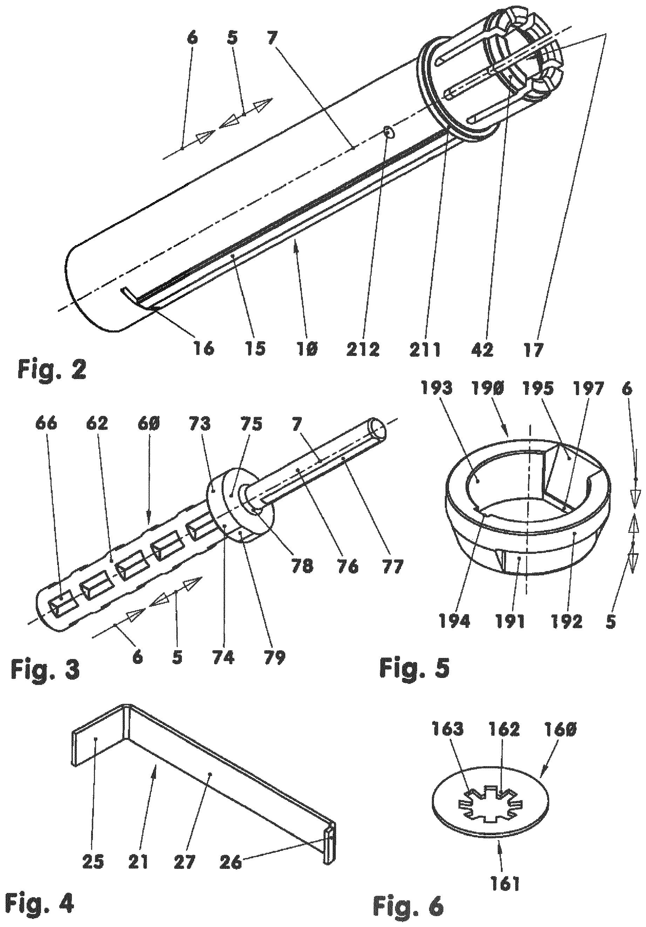

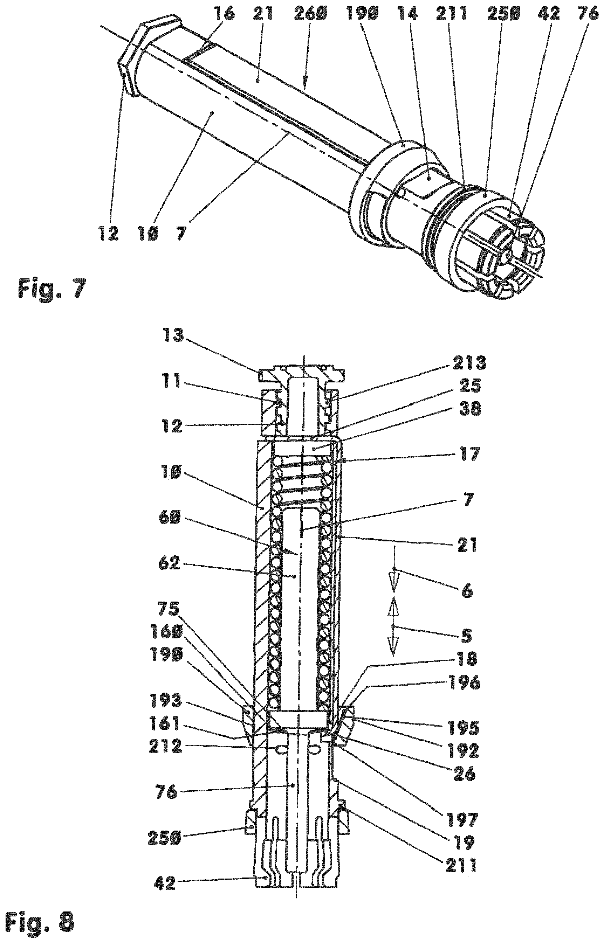

Das Gehäuse (10), vgl. auch Figur 2, hat z.B. eine Länge von 127 Millimetern. Es ist rohrförmig ausgebildet und hat eine weitgehend zylindrische Innenkontur. Der Innendurchmesser beträgt im Ausführungsbeispiel 15,5 Millimeter. Im hinteren, der Injektionsstelle abgewandten Bereich ist ein eingängiges Innengewinde (11) angeordnet, vgl. Figur 8. Die Außenkontur des Gehäuses (10) hat eine zylinderförmige Grundform mit einem Durchmesser von z.B. 20,5 Millimetern und mit einer Abflachung (14). Im Ausführungsbeispiel ist die Abflachung (14) mit dem hinteren Ende des Gehäuses (10) bündig und hat eine Länge von 100 Millimetern. In beiden Endbereichen der Abflachung (14) hat das Gehäuse (10) schlitzartige Durchbrüche (16) und (18), wobei der obere Durchbruch (16) das Gehäuse (10) durchdringt. Gegenüber der Abflachung (14) weist das Gehäuse (10) eine in der Längsrichtung (5) orientierte Führungsrippe (15) auf. Im vorderen, der Injektionsstelle zugewandten Bereich hat das Gehäuse (10) nach innen ragende Federhaken (42). Zwischen dem vorderen und dem hinteren Bereich ist eine ringförmig ausgebildete Gehäuseaufweitung (211) angeordnet. In der Nähe dieser Gehäuseaufweitung (211) sind im hinteren Bereich des Gehäuses (10) Montageöffnungen (212) angeordnet, die den Mantel des Gehäuses (10) durchdringen.

Der Federenergiespeicher (50) ist im Ausführungsbeispiel eine zylindrische Schraubendruckfeder (50) mit einem Außendurchmesser von z.B. 15,2 Millimetern. Sie ist aus austenitischem Stahl hergestellt und hat entlang ihrer Länge einen konstanten Drahtdurchmesser. Dieser beträgt im Ausführungsbeispiel 2,7 Millimeter. Die komprimierte Feder (50) hat eine Länge von 65 Millimetern. Im entspannten Zustand ist die Schraubendruckfeder (50) beispielsweise um mehr als 35 % länger als dieser Wert.

In der Figur 3 ist der Kolbenbetätigungsstempel (60) als Einzelteil dargestellt. Er umfasst einen z.B. zylindrischen Führungszapfen (62), einen Stempelteller (73) und einen Kolbenschieber (76). Der Kolbenbetätigungsstempel (60) ist beispielsweise aus einem glasfaserverstärkten Kunststoff hergestellt. Der eingesetzte thermoplastische Kunststoff ist z.B. ein Polyamid.

Der Führungszapfen (62) ist kürzer als die Länge der komprimierten Schraubendruckfeder (50). Er hat in radialer Richtung orientierte kreissegmentförmige Aussparungen (66). Diese sind beispielsweise am Umfang des Führungszapfens (62) und entlang seiner Länge verteilt. Im Ausführungsbeispiel ist hiermit die Masse des Führungszapfens (62) um 10 % niedriger als die Masse eines zylindrischen Führungszapfens ohne Aussparungen. Der Führungszapfen (62) kann auch rohrförmig ausgebildet sein.

Der Stempelteller (73) des Kolbenbetätigungsstempels (60) ist scheibenförmig ausgebildet und normal zur Mittellängsachse (7) des Einweginjektors (4) ausgerichtet. Sein Durchmesser beträgt beispielsweise 14,8 Millimeter. Er hat eine dem Führungszapfen (62) abgewandte, konisch ausgebildete Bundfläche (75). Der Spitzenwinkel des gedachten Kegels der Bundfläche (75) beträgt z.B. 160 Grad. An einander gegenüberliegenden Seiten der Umfangsfläche (74) hat der Stempelteller (73) jeweils eine Abflachung (79).

Der Kolbenschieber (76) ist stangenartig ausgebildet und hat im Ausführungsbeispiel einen Durchmesser von 5,2 Millimetern. Zwei einander gegenüberliegende, in Längsrichtung (5) orientierte Schlüsselflächen (77) haben jeweils eine Länge von z.B. 32 Millimetern. Die Bundfläche (75) des Stempeltellers (73) geht in einer umlaufenden Hohlkehle (78) in den Kolbenschieber (76) über.

Die Figur 4 zeigt den Zugstab (21), der aus einem Blechstreifen mit z.B. konstanter rechteckiger Querschnittsfläche hergestellt ist. Sein Werkstoff ist beispielsweise ein rost- und säurebeständiger Stahl, z.B. X10CrNi18-8 mit der Werkstoffnummer 1.4310. Dieser austenitische Werkstoff wird beispielsweise zur Herstellung von Federn eingesetzt. Sein Elastizitätsmodul ist z.B. größer als 190.000 Newton pro Quadratmillimeter. Die Länge des Zugstabes (21) beträgt im Ausführungsbeispiel 77 Millimeter, seine Breite 10 Millimeter und seine Dicke 1,2 Millimeter. Er ist u-förmig ausgebildet und umfasst einen Hauptschenkel (27), einen Klemmschenkel (25) und einen Umgriffshaken (26). Der z.B. mittels eines Biegeumformverfahrens abgewinkelte Klemmschenkel (25) schließt mit dem Hauptschenkel (27) einen Winkel von 90 Grad ein. Der Umgriffshaken (26) schließt mit dem Hauptschenkel (27) einen Winkel von 100 Grad ein. Die Länge des Umgriffshakens (26) beträgt beispielsweise 20 % der Länge des Klemmschenkels (25).

Der in der Figur 5 in einer dimetrischen Ansicht dargestellte Auslösering (190) ist im Ausführungsbeispiel aus einem glasfaserverstärkten thermoplastischen Kunststoff hergestellt. Dies ist z.B. Polyamid PA 6.6 mit 30 % Glasfaseranteil. Die Mantelfläche des Auslöserings (190) weist einen oberen zylinderförmigen Abschnitt (192) und einen unteren Abschnitt (191) auf. Der untere Abschnitt (191) ist bereichsweise zylindrisch und bereichsweise kegelstumpfförmig ausgebildet. Die in der Grundform zylinderförmige Innenwandung (193) hat auf der einen Seite eine in der Längsrichtung (5) orientierte Verdrehsicherungsnut (194). Auf der gegenüberliegenden Seite ist eine schräg ausgebildete Anlagefläche (195) ausgebildet. Diese Anlagefläche (195) ist beispielsweise in einem Winkel von 20 Grad zur Längsrichtung (5) ausgerichtet. Dieser Winkel, dessen Spitze in der Auslöserichtung (6) des Einweginjektors (4) versetzt zum Auslösering (190) liegt, kann zwischen 10 Grad und 45 Grad betragen. Die Anlagefläche (195) endet an einem unteren Absatz (197). Im Bereich dieses unteren Absatzes (197) ist die Innenwandung durch eine Sehne der Grundform begrenzt.

Bei der Montage wird eine Gehäuse-Vormontageeinheit (260), vgl. die Figuren 7 und 8 zusammengebaut. Hierbei wird beispielsweise zunächst eine Stützscheibe (160) auf den Kolbenschieber (76) des Kolbenbetätigungsstempels (60) aufgeschoben.

Im Ausführungsbeispiel ist die als Einzelteil in der Figur 6 dargestellte Stützscheibe (160) eine Lochscheibe. Sie ist beispielsweise aus dem gleichen Werkstoff wie der Zugstab (21) hergestellt. Um die zentrale Bohrung herum sind Nuten (163) und Keile (162) angeordnet, mit denen die Stützscheibe (160) nach dem Aufschieben formschlüssig auf dem Kolbenschieber (76) des Kolbenbetätigungsstempels (60) sitzt. In der Seitenansicht ist die Stützscheibe (160) kegelstumpfförmig ausgebildet. Der Spitzenwinkel entspricht dem Spitzenwinkel der Bundfläche (75), an der die Stützscheibe (160) nach dem Aufschieben auf den Kolbenbetätigungsstempel (60) anliegt.

Auf den Führungszapfen (62) des Kolbenbetätigungsstempels (60) wird die Schraubendruckfeder (50) aufgeschoben. Hierbei steht die entspannte Schraubendruckfeder (50) über den Kolbenbetätigungsstempel (62) über.

In das Gehäuse (10) wird eine Einstellscheibe (38) eingelegt. Die Dicke dieser zylindrischen Scheibe (38) bestimmt die Vorspannung des Einweginjektors (4). Je dicker die Einstellscheibe (38) ist, desto höher ist der Injektionsdruck des Einweginjektors (4). Gegebenenfalls kann die Gehäuse-Vormontageeinheit (260) auch ohne Einstellscheibe (38) ausgeführt sein.

In den oberen Gehäuseschlitz (16) wird der Klemmschenkel (25) des Zugstabs (21) eingeführt, sodass der Hauptschenkel (27) in Richtung der Gehäuseaufweitung (19) zeigt. Gegebenenfalls kann der Hauptschenkel (27) an der Abflachung (14) anliegen. Der Zugstab (21) ist damit asymmetrisch im Gehäuse (10) angeordnet.

Der Kolbenbetätigungsstempel (60) mit der Schraubendruckfeder (50) und dem Stützring (60) wird von der Seite der Federhaken (42) in den Innenraum (17) des Gehäuses (10) eingeführt. Die Schraubendruckfeder (50) liegt dann an der Einstellscheibe (38) an und diese kontaktiert den Klemmschenkel (25).

Der Auslösering (190) wird - vor oder nach dem Einschieben des Kolbenbetätigungsstempels (60) - von hinten her auf das Gehäuse (10) aufgeschoben. Hierbei ist in den Auslösering (190) eine Gleitplatte (196) eingelegt. Diese rechteckige Gleitplatte (196) ist z.B. aus dem gleichen Werkstoff wie der Zugstab (21) hergestellt und hat beispielsweise eine Dicke von 0,5 Millimetern. Sie liegt an der Anlagefläche (195) des Auslöserings (190) an und stützt sich am Absatz (197) ab. Gegebenenfalls kann sie dort fixiert sein.

Beim Aufschieben des Auslöserings (190) wird dieser am Zugstab (21) und an der Führungsrippe (15) des Gehäuses (10) geführt. Der Auslösering (190) wird so weit aufgeschoben, bis er an der Gehäuseaufweitung (19) unterhalb der rechteckigen Gehäuseaussparung (18) anliegt.

In das Innengewinde (11) des Gehäuses (10) wird eine Abstützschraube (12) eingeschraubt, bis sie am Klemmschenkel (25) anliegt oder gegen diesen presst. Die Abstützschraube (12) hat z.B. einen Sechskantabschnitt (13) zum Ansetzen eines Werkzeugs. Gegebenenfalls kann der Gewindegang (213) der Abstützschraube (12) und/oder des Gehäuses (10) eine Planverzahnung aufweisen, um ein unbeabsichtigtes Lösen der Abstützschraube (12) zu verhindern.

Nun wird der Kolbenbetätigungsstempel (60) z.B. mittels eines Werkzeugs in das Gehäuse (10) eingedrückt. Die Schraubendruckfeder (50) wird gespannt. Beispielsweise wird das Gehäuse (10) hierbei an einem Haltering (211) gehalten. Der Umgriffshaken (26) des Zugstabs (21) wird in die rechteckige Aussparung (18) eingeführt und an die Unterseite (161) der die Bundfläche (75) panzernden Stützscheibe (160) angelegt. Der Auslösering (190) wird nach oben gezogen, bis er am Zugstab (21) anliegt. Der Zugstab (21) stützt sich nun an der eine Panzerung des Auslöserings bildenden Gleitplatte (196) ab und wirkt als Sperrstab (21). Zur Sicherung der Montageposition kann ein z.B. u-förmiger Bügel in Montageöffnungen (212) des Gehäuses (10) eingeführt werden. Dieser Bügel sichert die Lage des Auslöserings (190) nach dem Abnehmen der Spannvorrichtung des Federenergiespeichers (50). Auch bei längerer Lagerung verursacht der federbelastete Zugstab (21) keine Verformungen der Gleitplatte (196). Damit ist auch nach längerer Lagerzeit ein sicheres Auslösen gewährleistet.

Im unteren Bereich des Gehäuses (10) kann ein Sicherungsring (250) auf die Federhaken (42) aufgeschoben werden, bis er beispielsweise am Haltering (211) anliegt. Hierbei werden die abgespreizten Federhaken (42) zusammengedrückt, sodass nach der Montage der Sicherungsring (250) mit einer Spielpassung verliersicher auf dem Gehäuse (10) sitzt. Diese Gehäuse-Vormontageeinheit (260) kann nun z.B. an einen weiteren Arbeitsplatz gefördert werden.

In einer anderen Fertigungslinie wird beispielsweise die Zylinder-Kolben-Einheit (100) hergestellt und befüllt. Die Zylinder-Kolben-Einheit (100) umfasst einen z.B. transparenten Zylinder (101) und einen im Zylinder (101) geführten Kolben (111). Im oberen Bereich hat der Zylinder (101) einen umlaufenden Bund (108). Die in der Stirnseite (103) angeordnete Austrittsöffnung (106) der Zylinder-Kolben-Einheit (100) ist als kurze, zylindrische, düsenartige Bohrung (106) ausgebildet. Der Durchmesser dieser Bohrung (106) beträgt beispielsweise 0,18 Millimeter.

Nach dem Befüllen des Zylinders (101) wird der Kolben (111) eingesetzt, der dann mit einer Presspassung im Zylinder (101) sitzt. Nun kann die Verschlusskappe (120) aufgesetzt werden. Diese weist an ihrer Umfangsfläche (122) eine Riffelung (123) auf, um ein Abrutschen der Finger zu vermeiden. Die so vorbereitete Zylinder-Kolben-Einheit (100) kann nun bis zur weiteren Montage gelagert werden.

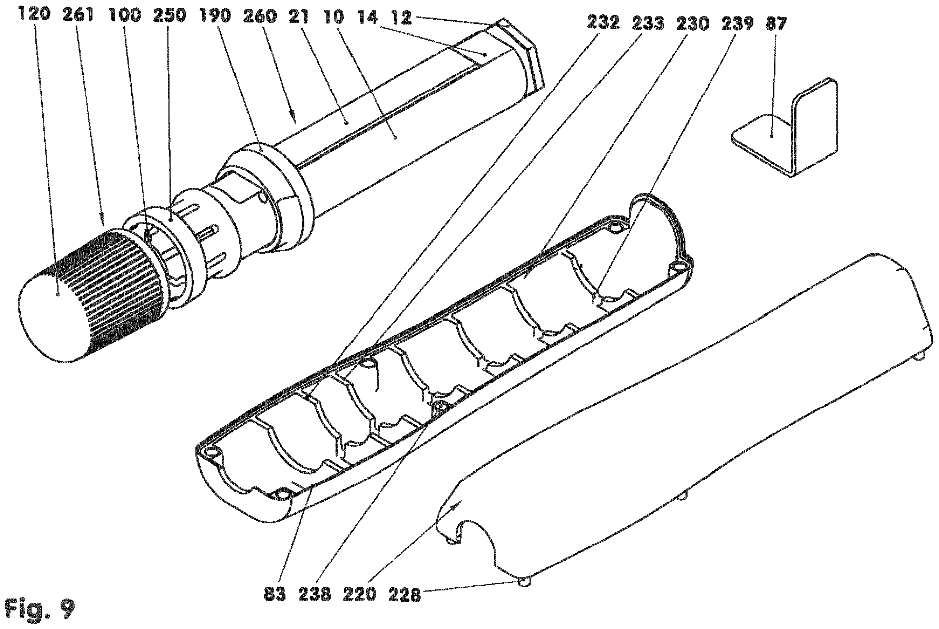

Vor der Endmontage wird die Zylinder-Kolben-Einheit (100) in die Gehäuse-Vormontagegruppe (260) eingesetzt, vgl. Figur 9. Hierbei wird die Zylinder-Kolben-Einheit (100) unter Aufweitung der Federhaken (42) so weit in das Gehäuse (10) eingeschoben, bis die Federhaken (42) den Bund (108) hintergreifen und verrasten. Die Federhaken (42) wirken als Rasthaken (42). Nun wird der Sicherungsring (250) nach vorne geschoben und presst die Rasthaken (42) gegen die Zylinder-Kolben-Einheit (100). Die Zylinder-Kolben-Einheit (100) ist jetzt verliersicher im Gehäuse (10) gehalten. Die Verschlusskappe (120) ist vom Gehäuse (10) beabstandet. Diese Baugruppe (261) kann behandelt, transportiert und gelagert werden.

Das Mantelgehäuse (82) besteht im Ausführungsbeispiel aus einer Oberschale (220) und einer Unterschale (230). Die Trennfuge (83) beider Schalen (220, 230) ist in der Längsrichtung (5) des Einweginjektors (4) orientiert.

Die beiden Schalen (220, 230) des Mantelgehäuses (82) haben an ihrer Innenseite jeweils Verstärkungsrippen (221 - 227; 231 - 237). Diese Querrippen (221 - 227; 231 - 237) sind normal zur Längsrichtung (5) des Einmalinjektors (4) orientiert. Hierbei weisen die hinteren Verstärkungsrippen (223 - 227; 232 - 237) jeweils zwei Teile auf, zwischen denen eine Nut (239) angeordnet ist. Die dem Bediener abgewandten vordersten Verstärkungsrippen (221, 222; 231) sind jeweils unterbrechungsfrei ausgeführt. Die Oberschale (220) und die Unterschale (230) werden beispielsweise mittels mehrerer Zapfenverbindungen (228, 238) miteinander verbunden. Hierbei weist im Ausführungsbeispiel die Oberschale (220) an der Trennfuge (83) sechs Zapfen (228) auf, die in Zapfenlöcher (238) der Unterschale (230) eingreifen. Gegebenenfalls können die Zapfenverbindungen (228, 238) bei der Montage miteinander verrasten. Auch ein Verkleben der Unterschale (230) mit der Oberschale (220) ist denkbar.

Das Gehäuse (10) und das Mantelgehäuse (82) sind beispielsweise aus Kunststoff hergestellt. Dies kann ein thermo- oder duroplastischer Werkstoff, z.B. POM, ABS, etc. sein.

Bei der Endmontage des Einweginjektors (4) wird die Gehäuse-Vormontagegruppe (260) mit der Zylinder-Kolben-Einheit (100) beispielsweise in die Unterschale (230) eingelegt. Hierbei wird die Rippe (15) des Gehäuses (10) in der Längsnut (239) der Unterschale (230) zentriert. Der Auslösering (190) liegt zwischen den zweiten Querrippen (222, 232) und den dritten Querrippen (223, 233). Der Kopf der Abstützschraube (12) ragt über die hinterste Querrippe (227; 237) heraus. Die Verschlusskappe (120) sitzt außerhalb des Mantelgehäuses (82). In den Schlitz (241) wird das Sicherungselement (87) eingesetzt, das beispielsweise zwischen der Sicherungsschraube (12) und dem Mantelgehäuse (82) geklemmt wird. Die Abstützschraube (12) kann gegen weiteres Verdrehen z.B. formschlüssig gesichert werden. Gegebenenfalls kann eine zusätzliche Druckfeder zwischen der Abstützschraube (12) und dem Mantelgehäuse (82) den Widerstand gegen versehentliches Auslösen erhöhen. Diese Feder bestimmt auch den Widerstand des Einweginjektors (4) beim Auslösen. Der u-förmige Bügel kann entfernt werden.

Zum Abschluss der Montage wird die Oberschale (220) auf die Unterschale (230) aufgesetzt und z.B. durch Verkleben, Verrasten, etc. gesichert. Nun kann ein zusätzlicher Originalitätsverschluss, z.B. eine Banderole, über das Mantelgehäuse (82) und die Verschlusskappe (120) angebracht werden. Es ist auch denkbar, die Montage in einer anderen als in der beschriebenen Reihenfolge durchzuführen.

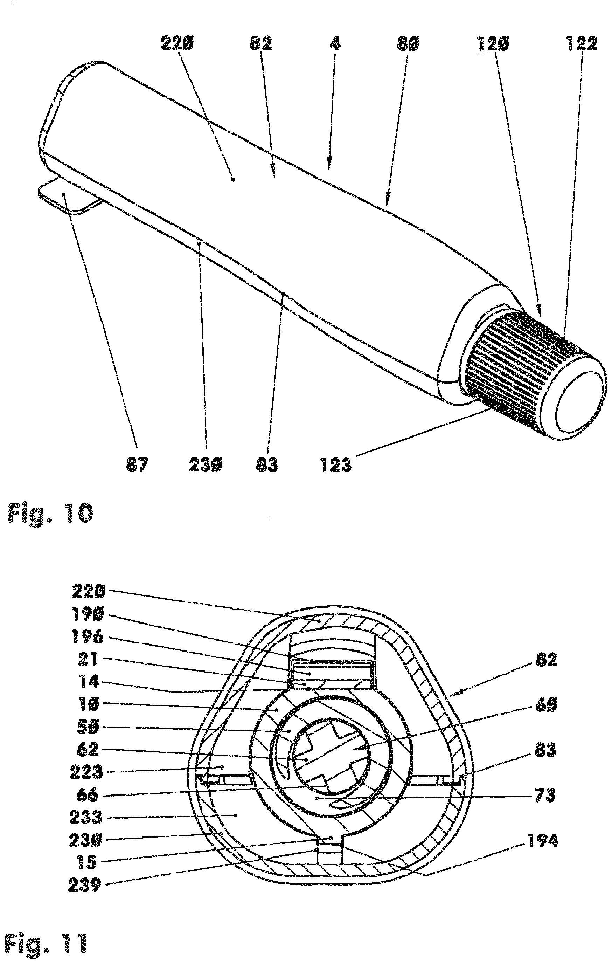

Der fertig montierte Einweginjektor (4) ist in der Figur 10 in einer dimetrischen Ansicht und in der Figur 11 in einem Querschnitt dargestellt. Der Querschnitt des Mantelgehäuses (82) hat keine Ecken und Kanten und weicht von einer Kreisform ab. Im Ausführungsbeispiel ist der Querschnitt ähnlich einem regelmäßigen Dreieck mit abgerundeten Ecken ausgebildet. Es ist aber auch denkbar, den Querschnitt polygonähnlich auszubilden.

Die Querschnittsfläche im hinteren Bereich des Einweginjektors (4) beträgt 70 % der Querschnittsfläche im vorderen, der Injektionsstelle zugewandten Bereich des Einweginjektors (4). Der stetige Anstieg der Querschnittsfläche liegt, von hinten her gesehen, im dritten Viertel der Länge des Einweginjektors (4). Damit ist der Einweginjektor (4) für den Bediener ohne Schwierigkeiten greifbar. Beispielsweise umgreift er das Mantelgehäuse (82) mit allen Fingern. Hierbei kann die Austrittsöffnung (106) z.B. auf der Seite des Zeigefingers oder auf der Seite des kleinen Fingers liegen.

Der fertig montierte Einweginjektor (4) kann nun verpackt und vertrieben werden. Wird er z.B. nach dem Auspacken auf einem Tisch abgelegt, besteht aufgrund der Gehäusegeometrie keine Gefahr des Wegrollens.

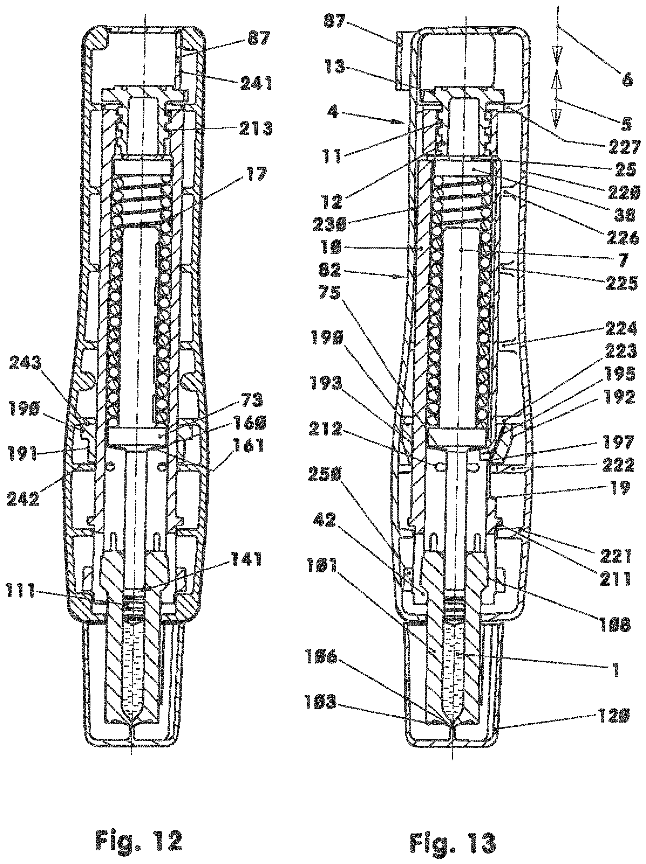

Der in den Figuren 12 und 13 in zueinander normal orientierten Längsschnitten dargestellte Einweginjektor (4) umfasst das Mantelgehäuse (82), in dem das innere Gehäuse (10) mit dem Kolbenbetätigungsstempel (60) und der Schraubendruckfeder (50) als dauergeladener Federenergiespeicher (50) sowie die Zylinder-Kolben-Einheit (100) angeordnet sind. Der Kolbenbetätigungsstempel (76) ist mittels eines Zwischenraums (141) vom Kolben (111) beabstandet. Die Zylinder-Kolben-Einheit (100) ist mittels der Schutzkappe (120) verschlossen. Das Auslösen des Einmalinjektors (4) wird mittels des Sicherungsschiebers (87) verhindert. Hierbei bildet die dem Auslösering (190) zugewandte Seite der Querrippen (222, 232) eine Sperrflanke (242).

Vor dem Einsatz des Einmalinjektors (4) wird zunächst der Originalitätsverschluss entfernt. Nach dem Abnehmen der Verschlusskappe (120) kann das Sicherungselement (87) herausgezogen werden. Der Einmalinjektor (4) ist nun einsatzbereit und wird beispielsweise auf die Haut des Patienten aufgesetzt. Auch in diesem Zustand verhindert die Selbsthemmung zwischen dem Sperrstab (21) und dem Auslösering (190) ein unbeabsichtigtes Selbstauslösen des Einweginjektors (4).

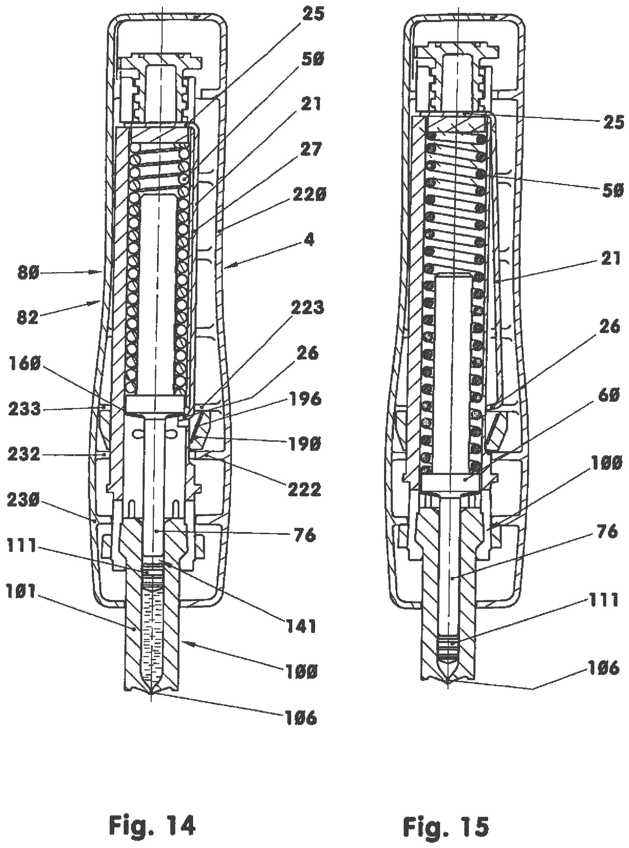

Zum Auslösen des Einmalinjektors (4) wird vom Bediener die Auslösevorrichtung (80) betätigt. Der Bediener drückt das Mantelgehäuse (82), das ein Auslöseelement in der Bauform einer Auslösehülse (82) bildet, in der Auslöserichtung (6) relativ zum Gehäuse (10) nach vorne, also in Richtung der Haut des Patienten. Die Auslösehülse (82) verschiebt hierbei mittels der dem Auslösering (190) zugewandten Seite der Querrippe (223, 233), die eine Auslöseflanke (243) bildet, den Auslösering (190) relativ zum Gehäuse (10) nach unten, vgl. die Schnittdarstellungen der Figuren 12 - 15. In der Figur 14 ist der nichtstatische Zustand unmittelbar nach dem Auslösen dargestellt. Der Federenergiespeicher (50) drückt den Stempelteller (73) nach vorne. Hierbei verdrängt die Druckscheibe (160) den Umgriffshaken (26) des Sperrstabs (21). Der Sperrstab (21) gleitet entlang der Gleitplatte (196) nach außen und gibt damit den Kolbenbetätigungsstempel (60) vollständig frei. Hierbei kann der Sperrstab (21) gegebenenfalls auf eine dämmende Gummischicht auftreffen. Der Kolbenbetätigungsstempel (60) schnellt, belastet von dem sich entspannenden Federenergiespeicher (50), nach vorne bzw. unten. Der Kolbenschieber (76) schlägt auf den Kolben (111) und schiebt diesen nach vorne. Hierbei wird die Luft aus dem Zwischenraum (141) entlang der Schlüsselflächen (77) verdrängt. Die im Zylinder (101) gelagerte Injektionslösung (1) wird durch die Austrittsöffnung (106) und die Hornhaut des Patienten hindurch in den Körper des Patienten verdrängt.

Die Figur 15 zeigt den Einmalinjektor (4) nach dem Auslösen. Die Auslösevorrichtung (80) und der Auslösering (190) sind relativ zum Gehäuse (10) nach unten verschoben. Der Anschlag der Auslösehülse (82) am Sicherungsring (250) verhindert ein weiteres Ausschieben der Auslösevorrichtung (80) gegenüber dem Gehäuse (10). Der Sperrstab (21) ist nach außen verdrängt. Hierbei blockiert er z.B. das Wiedereinschieben der Auslösehülse (82) mit dem Auslösering (190). Der Federenergiespeicher (50) ist entspannt. Der Kolbenbetätigungsstempel (60) liegt in seiner vorderen Endlage. Die Zylinder-Kolben-Einheit (100) ist entleert.

Selbstverständlich ist es auch denkbar, die verschiedenen genannten Ausführungsformen miteinander zu kombinieren.

### Bezugszeichenliste:

- 1: Injektionslösung, Wasser für Injektionszwecke
- 4: Einmalinjektor, Einweginjektor
- 5: Längsrichtung
- 6: Auslöserichtung
- 7: Mittellängsachse

- 10: Gehäuse
- 11: Innengewinde
- 12: Abstützschraube
- 13: Sechskantabschnitt
- 14: Abflachung
- 15: Führungsrippe
- 16: Gehäuseöffnung, schlitzförmig
- 17: Innenraum
- 18: Gehäuseöffnung mit rechteckigem Querschnitt
- 19: Gehäuseaufweitung

- 21: Zugstab, Sperrstab

- 25: Klemmschenkel
- 26: Umgriffshaken
- 27: Hauptschenkel

- 38: Scheibe, Einstellscheibe
- 42: Rasthaken, Federhaken

- 50: Federenergiespeicher, Schraubendruckfeder, Feder

- 60: Kolbenbetätigungsstempel

- 62: Führungszapfen
- 66: Aussparungen

- 73: Stempelteller
- 74: Umfangsfläche
- 75: Bundfläche
- 76: Kolbenschieber
- 77: Schlüsselflächen
- 78: Hohlkehle
- 79: Abflachung

- 80: Auslösevorrichtung
- 82: Mantelgehäuse, Auslöseelement, Auslösehülse
- 83: Trennfuge

- 87: Sicherungsschieber, Sicherungselement

- 100: Zylinder-Kolben-Einheit
- 101: Zylinder
- 103: Stirnfläche
- 106: Bohrung, Austrittsöffnung
- 108: Bund

- 111: Kolben
- 120: Verschlusskappe

- 122: Umfangsfläche
- 123: Riffelung

- 141: Zwischenraum

- 160: Stützscheibe, Druckscheibe
- 161: Unterseite
- 162: Keile
- 163: Nuten

- 190: Auslösering
- 191: unterer Bereich
- 192: zylinderförmiger Bereich
- 193: Innenwandung
- 194: Verdrehsicherungsnut
- 195: Anlagefläche
- 196: Einschubblech, Gleitplatte
- 197: Absatz

- 211: Haltering
- 212: Montageöffnungen, Sicherungselement-Durchbrüche
- 213: Gewindegang

- 220: Oberschale
- 221, 222: Verstärkungsrippen, Querrippen, einteilig
- 223 - 227: Verstärkungsrippen, Querrippen, zweiteilig
- 228: Zapfen

- 230: Unterschale
- 231: Verstärkungsrippe, Querrippe, einteilig
- 232 - 237: Verstärkungsrippe, Querrippe, zweiteilig
- 238: Zapfenlöcher
- 239: Längsnut

- 241: Schlitz
- 242: Sperrflanke
- 243: Auslöseflanke

- 250: Sicherungsring

- 260: Gehäuse-Vormontageeinheit, Gehäuse-Vormontagegruppe
- 261: Baugruppe

## Patentansprüche

1. Einweginjektor (4) mit einem in einem Gehäuse (10) gelagerten, mittels eines Federenergiespeichers (50) belasteten und mittels einer verschiebbaren Auslösevorrichtung (80) entsperrbaren Kolbenbetätigungsstempel (60), wobei der Kolbenbetätigungsstempel (60) mittels eines im Gehäuse (10) gelagerten Zugstabs (21) abstützbar ist,
- wobei die Auslösevorrichtung (80) ein Mantelgehäuse (82) umfasst,
- wobei das Mantelgehäuse (82) eine Oberschale (220) und eine Unterschale (230) umfasst, deren Trennfuge (83) in der Längsrichtung (5) des Einweginjektors (4) orientiert ist,
- wobei das Mantelgehäuse (82) einen von der kreisförmigen Form abweichenden eckenlosen Querschnitt aufweist,
- wobei das Gehäuse (10) im Mantelgehäuse (82) verdrehsicher zentriert ist, und
- wobei der Kolbenbetätigungsstempel (60) einen Führungszapfen (62), einen Stempelteller (73) und einen Kolbenschieber (76) umfasst, **dadurch gekennzeichnet,**
- **dass** der Stempelteller (73) eine dem Führungszapfen (62) abgewandte, konisch ausgebildete Bundfläche (75) hat, auf der eine kegelstumpfförmig ausgebildete Stützscheibe (160) aufliegt, deren Spitzenwinkel dem Spitzenwinkel der Bundfläche (75) entspricht,
- **dass** der Zugstab (21) u-förmig und streifenförmig ausgebildet ist und einen Hauptschenkel (27), einen Klemmschenkel (25) und einen Umgriffsschenkel (26) umfasst,
- **dass** der Zugstab (21) mittels des Klemmschenkels (25) im Gehäuse (10) gelagert ist
- **dass** der Umgriffsschenkel (26) durch einen schlitzartigen Durchbruch (18) des Gehäuses (10) hindurchragt und an einer Unterseite (161) der Stützscheibe (160) anliegt,
- **dass** sich der Zugstab (21) an einer eine Panzerung bildenden Gleitplatte (196) eines Auslöserings (190) abstützt,
- **dass** der Auslösering (190) das Gehäuse (10) umgreift und zwischen einer Sperrflanke (242) und einer Auslöseflanke (243) des Mantelgehäuses (82) angeordnet ist,
- **dass** beim Auslösen das eine Auslösehülse (82) bildende Mantelgehäuse (82) mittels der Auslöseflanke (243) den Auslösering (190) relativ zum Gehäuse (10) in der Auslöserichtung (6) nach vorne verschiebt,
- **dass** der Federenergiespeicher (50) den Stempelteller (73) nach vorne drückt und die Stützscheibe (160) den Umgriffshaken (26) des Zugstabs (21) verdrängt,
- sodass der Zugstab (21) entlang der Gleitplatte (196) nach außen gleitet und den Kolbenbetätigungsstempel (60) vollständig freigibt.

2. Einweginjektor (4) nach Anspruch 1,
**dadurch gekennzeichnet,**
**dass** im Mantelgehäuse (82) eine Vormontageeinheit (260) angeordnet ist, die das Gehäuse (10) mit dem Federenergiespeicher (50), dem Kolbenbetätigungsstempel (60), dem Zugstab (21) und dem Auslösering (190) umfasst.

3. Einweginjektor (4) nach Anspruch 1,
**dadurch gekennzeichnet,**
**dass** der Zugstab (21) asymmetrisch im Gehäuse (10) angeordnet ist.

4. Einweginjektor (4) nach Anspruch 1,
**dadurch gekennzeichnet,**
**dass** das Gehäuse (10) ein Innengewinde (11) aufweist, in dem eine den Zugstab (21) klemmende Abstützschraube (12) eingeschraubt ist.

5. Einweginjektor (4) nach Anspruch 1,
**dadurch gekennzeichnet,**
**dass** das Gehäuse (10) Sicherungselement-Durchbrüche (212) aufweist.

6. Einweginjektor (4) nach Anspruch 1,
**dadurch gekennzeichnet,**
**dass** er eine Zylinder-Kolben-Einheit (100) umfasst, die mittels Rasthaken (42) und einem diese umgebenen Sicherungsring (250) im Gehäuse (10) gesichert angeordnet ist.

7. Einweginjektor (4) nach Anspruch 1,
**dadurch gekennzeichnet,**
**dass** zwischen dem Federenergiespeicher (50) und dem Gehäuse (10) eine Einstellscheibe (38) angeordnet ist.

8. Einweginjektor (4) nach Anspruch 1,
**dadurch gekennzeichnet,**
**dass** das Gehäuse (10) mittels eines entriegelbaren Sicherungselements (87) im Mantelgehäuse (82) abgestützt ist.

## Claims

1. A single-use injector (4) having a piston actuation ram (60) which is mounted in a housing (10) and which is loaded by means of a spring energy store (50) and which can be released by means of a displaceable triggering device (80), wherein the piston actuation ram (60) can be supported by means of a tension bar (21) which is mounted in the housing (10),
- wherein the triggering device (80) comprises a covering housing (82),
- wherein the covering housing (82) comprises an upper shell (220) and a lower shell (230), the separation joint (83) of which is orientated in the longitudinal direction (5) of the single-use injector (4),
- wherein the covering housing (82) has a cornerless cross-section which differs from a circular shape,
- wherein the housing (10) is centred in the covering housing (82) in a manner secured against rotation, and
- wherein the piston actuation ram (60) has a guide journal (62), a ram plate (73), and a piston slider (76), **characterised in that**
- the ram plate (73) has a conical collar face (75) which faces away from the guide journal (62), with a frustoconical support disc (160) resting on said collar face, the apex angle of said support disc corresponding to the apex angle of the collar face (75),
- the tension bar (21) is U-shaped and strip-like and comprises a main limb (27), a clamping limb (25), and a wrap-around limb (26),
- **in that** the tension bar (21) is mounted in the housing (10) by means of the clamping limb (25),
- **in that** the wrap-around limb (26) protrudes through a slot-like aperture (18) in the housing (10) and bears against an underside (161) of the support disc (160),
- **in that** the tension bar (21) is supported on a sliding plate (196) of a triggering ring (190), which siding plate forms an armour-plating,
- **in that** the triggering ring (190) runs around the housing (10) and is arranged between a locking flank (242) and a triggering flank (243) of the covering housing (82),
- **in that**, during the triggering, the covering housing (82) forming a triggering sleeve (82) shifts the triggering ring (190) forward relative to the housing (10) in the triggering direction (6) by means of the triggering flank (243),
- **in that** the spring energy store (50) pushes the ram plate (73) forward and the support disc (160) displaces the wrap-around hook (26) of the tension bar (21),
- such that the tension bar (21) slides outwardly along the sliding plate (196) and completely frees the piston actuation ram (60).

2. The single-use injector (4) according to claim 1, **characterised in that** a preassembly unit (260) is arranged in the covering housing (82) and comprises the housing (10) with the spring energy store (50), the piston actuation ram (60), the tension bar (21) and the triggering ring (190).

3. The single-use injector (4) according to claim 1, **characterised in that** the tension bar (21) is arranged asymmetrically in the housing (10).

4. The single-use injector (4) according to claim 1, **characterised in that** the housing (10) has an inner thread (11), in which a support screw (12) which clamps the tension bar (21) is screwed.

5. The single-use injector (4) according to claim 1, **characterised in that** the housing (10) has securing element apertures (212).

6. The single-use injector (4) according to claim 1, **characterised in that** it comprises a cylinder-piston unit (100) which is arranged in a state secured in the housing (10) by means of detent hooks (42) and a securing ring (250) which surrounds them.

7. The single-use injector (4) according to claim 1, **characterised in that** an adjustment disc (38) is arranged between the spring energy store (50) and the housing (10).

8. The single-use injector (4) according to claim 1, **characterised in that** the housing (10) is supported in the covering housing (82) by means of an unlockable securing element (87).

## Revendications

1. Injecteur à usage unique (4), comprenant un poinçon d'actionnement de piston (60) logé dans un boîtier (10) qui est sollicité par un accumulateur d'énergie à ressort (50) et qui peut être débloqué à l'aide d'un dispositif de déclenchement coulissant (80), le poinçon d'actionnement du piston (60) pouvant être soutenu à l'aide d'une tige de traction (21) montée dans le boîtier (10),
- le dispositif de déclenchement (80) comprenant un boîtier enveloppant (82),
- le boîtier enveloppant (82) comprenant une coque supérieure (220) et une coque inférieure (230) dont le joint de séparation (83) est orienté dans le sens longitudinal (5) de l'injecteur à usage unique (4),
- le boîtier enveloppant (82) présentant une section exempte de coins qui s'écarte de la forme circulaire du boîtier,
- le boîtier (10) étant centré de manière fixe en rotation dans le boîtier enveloppant (82), et
- le poinçon d'actionnement du piston (60) comprenant un tenon de guidage (62), une plaque d'appui (73) et une tige poussoir de piston (76), **caractérisé en ce**
- **que** la plaque d'appui (73) possède une surface supérieure conique (75) opposée au tenon de guidage (62) sur laquelle repose une rondelle d'appui de forme tronconique (160) dont l'angle de pointe correspond à l'angle de pointe de la surface supérieure (75),
- **que** la tige de traction (21) est en forme de U et de bande et qu'elle comprend une branche principale (27), une branche de serrage (25) et une branche formant un crochet (26),
- **que** la tige de traction (21) est montée dans le boîtier au moyen de la branche de serrage (25),
- **que** le crochet (26) passe au travers d'une ouverture en forme de fente (18) du boîtier (10) et s'applique contre un côté inférieur de la rondelle d'appui (160),
- **que** la tige de traction (21) s'appuie sur une plaque coulissante (196) d'une bague de déclenchement (190) formant un blindage,
- **que** la bague de déclenchement (190) entoure le boîtier (10) et est disposée entre un flanc de blocage (242) et un flanc de déclenchement (243) du boîtier enveloppant (82),
- **que** le boîtier enveloppant (82) qui forme un manchon de libération (82) déplace la bague de déclenchement (190) au moyen du flanc de déclenchement (243) lors du déclenchement vers l'avant, dans la direction du déclenchement (6) par rapport au boîtier (10),
- **que** l'accumulateur d'énergie à ressort (50) pousse la plaque d'appui (73) vers l'avant et que la rondelle d'appui (160) chasse le crochet (26) de la tige de traction (21) de telle façon
- **que** la tige de traction (21) glisse le long de la plaque coulissante (196) vers l'extérieur et libère le poinçon d'actionnement du piston (60) complètement.

2. Injecteur à usage unique (4) selon la revendication 1,
**caractérisé en ce**
**qu'**une unité de pré-assemblage (260) qui comprend le boîtier (10) avec l'accumulateur d'énergie à ressort (50), le poinçon d'actionnement de piston (60), la tige de traction (21) et la bague de déclenchement (190), est logée dans le boîtier enveloppant (82).

3. Injecteur à usage unique (4) selon la revendication 1,
**caractérisé en ce**
**que** la tige de traction (21) est placée dans le boîtier (10) de façon asymétrique.

4. Injecteur à usage unique (4) selon la revendication 1,
**caractérisé en ce**
**que** le boîtier (10) présente un filetage intérieur (11) dans lequel est vissée une vis d'appui (12) serrant la tige de traction (21).

5. Injecteur à usage unique (4) selon la revendication 1,
**caractérisé en ce**
**que** le boîtier (10) présente des ouvertures de fixation (212).

6. Injecteur à usage unique (4) selon la revendication 1,
**caractérisé en ce**
**qu'**il comprend une unité cylindre-piston (100) qui est placée dans le boîtier de manière sécurisée par des crochets d'encliquetage (42) et un anneau de sécurité (250) entourant les dits crochets.

7. Injecteur à usage unique (4) selon la revendication 1,
**caractérisé en ce**
**qu'**une vis de réglage (38) est positionnée entre l'accumulateur d'énergie à ressort (50) et le boîtier (10).

8. Injecteur à usage unique (4) selon la revendication 1,
**caractérisé en ce**
**que** le boîtier (10) est soutenu dans le boîtier enveloppant (82) à l'aide d'un élément de sécurité déverrouillable (87).
